# EUROPÄISCHE PATENTSCHRIFT

(11) **EP 1 593 357 B1**
(45) Veröffentlichungstag und Bekanntmachung des Hinweises auf die Patenterteilung: **04.01.2017**
(21) Anmeldenummer: 05009856.5
(22) Anmeldetag: 04.05.2005
(51) Int. Cl.: A61F 2/44

(54) **Flexibler Platzhalter**
Flexible spacer
Ecarteur flexible

(30) Priorität: 04.05.2004 DE 102004021861; 04.05.2004 US 567989 P
(43) Veröffentlichungstag der Anmeldung: 09.11.2005
(60) Teilanmeldung: 08103566.9
(73) Patentinhaber: Biedermann Technologies GmbH & Co. KG, 78166 Donaueschingen (DE)
(72) Erfinder: Matthis, Wilfried, 79367 Weisweil (DE); Biedermann, Lutz, 78048 VS-Villingen (DE); Harms, Jürgen, Prof., 76227 Karlsruhe (DE)
(74) Vertreter: Lang, Christian

(56) Entgegenhaltungen:
- EP-A- 0 529 275
- EP-A- 0 950 389
- EP-A- 1 273 276
- WO-A-99/65425
- WO-A1-2004/019827
- DE-U1- 20 213 013
- FR-A- 2 734 148
- US-A- 5 423 816
- US-A- 5 423 817
- US-A1- 2004 002 761
- US-B1- 6 579 321

## Beschreibung

Die Erfindung betrifft einen flexiblen Platzhalter nach dem Oberbegriff des Anspruchs 1.

In der modernen Medizin können viele Defekte am menschlichen oder tierischen Körper durch den Einsatz von Implantaten ausgeglichen oder in ihren Wirkungen minimiert werden. Beispielsweise sind Platzhalter für Wirbel oder Bandscheiben bekannt, die zum Ersatz eines Wirbelkörpers oder einer Bandscheibe dienen.

Bei den Platzhaltern kommt es darauf an, dass Werkstoffe zum Einsatz kommen, die mit dem menschlichen oder tierischen Organismus verträglich sind, also keine Abstoßreaktionen hervorrufen oder durch Auflösungserscheinungen zu einer Belastung des Organismus führen. Entsprechend ist die Auswahl an Werkstoffen für Platzhalter deutlich eingeschränkt.

Darüber hinaus ist es vorteilhaft, die Platzhalter möglichst einfach und insbesondere aus wenigen Teilen zu bilden, da das Zusammensetzen der Teile einen erhöhten Aufwand für den Operateur beim Einsetzen der Implantate begründet und zum anderen durch die Verbindungsstellen der einzelnen Teile zueinander eine größere Fehleranfälligkeit und damit Wahrscheinlichkeit für Fehlfunktionen gegeben ist. Insofern ist es besonders bevorzugt, Platzhalter einstückig auszubilden.

Demgegenüber müssen Platzhalter jedoch unterschiedliche Funktionen erfüllen, die es wünschenswert erscheinen lassen, unterschiedliche Werkstoffe einzusetzen und/oder Platzhalter aus mehreren Bauteilen zusammenzusetzen. Beispielsweise ist es für Platzhalter erstrebenswert, dass sie nicht nur die Funktion des Ausfüllens des Platzes und Haltens der Wirbel in einem bestimmten Abstand zueinander erfüllen, sondern dass sie darüber hinaus eine gewisse Beweglichkeit der Wirbel zueinander ermöglichen, also in gewissen engen Grenzen eine Gelenkfunktion ausfüllen. Zu diesem Zweck ist es beispielsweise möglich, einen Platzhalter gemäß der DE 10056977 C2 vorzusehen, bei dem zwischen den an den Wirbelkörpern angelegten Stützelementen ein balgförmiges, in Längsrichtung des Implantats verlängerbares Schlauchstück aus einem dicht gewebten oder gewirkten Textilmaterial angeordnet wird. Dies hat jedoch den oben beschriebenen Nachteil, dass mehrere unterschiedliche Werkstoffe eingesetzt werden müssen, die miteinander verbunden werden müssen, was die Fehleranfälligkeit erhöhen kann.

Aus den Dokumenten US 5,423,816 A und WO 99/65425 A2 sind Wirbelsäulenimplantate bekannt, bei denen innerhalb von Stützkörpern mit spiralförmigen Aussparungen Knochenspäne angeordnet wird. Aus der D2 ist zudem bekannt ein Wirbelsäulenimplantat mit Endplatten vorzusehen, die bei Belastung aneinander anliegen und zur Kraftübertragung eingesetzt werden können.

DE 202 13 013 U1 offenbart einen Platzhalter für Wirbel und/oder Bandscheiben mit Platzhalter- und Gewichtsübertragungsfunktion mit einem zylinderrohrartigen Körper und mit an den stirnseitigen Enden des zylinderrohrartigen Körpers vorgesehenen Mitteln zum Verbinden mit benachbarten Körperteilen, wobei im zylinderrohrartigen Körper Materialaussparungen vorgesehen sind, wobei der zylinderrohrartige Körper von einer Hülse umgeben oder/ und mit einem bestehenden Kernelement versehen ist, welche durch an dem zylinderrohrartigen Körper einstückig und/oder lösbar, insbesondere über eine Schraub-oder Gewindeverbindung, angeordnete Endplatten gehalten werden.

Es ist deshalb Aufgabe der vorliegenden Erfindung, Platzhalter bereitzustellen, die möglichst einfach, insbesondere einstückig oder aus wenigen einfach zu verbindenden Teilen aufgebaut sind und bei denen neben der Platzhalter- und Gewichtsübertragungsfunktion eine gewisse Flexibilität und Beweglichkeit innerhalb des Platzhalters bzw. Bereichen davon gewährleistet werden soll. Darüber hinaus sollen diese Platzhalter einfach herstellbar und implantierbar und sicher im Betrieb sein sowie eine lange Lebensdauer aufweisen.

Diese Aufgabe wird gelöst durch Platzhalter mit den Merkmalen gemäß Anspruch 1. Vorteilhafte Ausgestaltungen sind Gegenstand der abhängigen Ansprüche.

Bei den Platzhaltern wird nach dem Stand der Technik die Beweglichkeit oder Elastizität durch zusätzliche Bauteile oder Werkstoffe erreicht. Die Erfindung geht hierbei einen anderen Weg, indem die Flexibilität bzw. Beweglichkeit primär nicht durch einen anderen Werkstoff oder durch das Vorsehen von zusätzlichen getrennten Bauteilen bewirkt wird, sondern dass dies durch das konstruktive Vorsehen von Materialaussparungen erreicht wird.

Erfindungsgemäß weisen die Platzhalter einen zylinderrohrartigen Körper mit einem mittleren zylinderrohrartigen Körperteil sowie an den Stirnseiten vorgesehene Verbindungselemente auf, wobei die für die Flexibilität verantwortlichen Materialaussparungen im zylinderrohrartigen Körperteil vorgesehen sind.

Die Verbindungselemente der Platzhalter weisen vorzugsweise entsprechende Mittel zum Verbinden des Platzhalters mit benachbarten Körperteilen, wie z. B. Wirbeln, in Form von zackenartigen Vorsprüngen an den Stirnseiten und/oder Aussparungen, Vertiefungen und Öffnungen an der Mantelfläche zum Einwachsen und Verwachsen des Platzhalters mit dem Gewebe auf. Hierbei dürfen jedoch die Aussparungen oder Vertiefungen der Verbindungselemente nicht mit den Materialaussparungen zur Erzielung einer Flexibilität und Beweglichkeit des Platzhalters im zylinderrohrartigen Körperteil verwechselt werden. Da die Verbindungselemente mit den benachbarten Körperteilen, wie den Wirbeln, vollständig verwachsen, tragen diese nichts zur Flexibilität bzw. Beweglichkeit der Wirbel zueinander bei.

Auf die erfindungsgemäße Weise kann in einfacher Weise bei Verzicht auf zusätzliche elastische Werkstoffe und entsprechende Verbindungsstellen oder zusätzliche getrennte Bauteile eine Flexibilität und Beweglichkeit innerhalb des Platzhalters oder Teilen davon erreicht werden. Hierbei können die Elastizitäts- oder Beweglichkeitsfunktionen zusätzlich zu den notwendigen Funktionen des Platzhalters vorgesehen werden. Insbesondere können auf diese Weise Kompressions-, Torsions- und/oder Dehnzonen in einfacher und zuverlässiger Weise insbesondere in einem einstückigen Platzhalter verwirklicht werden.

Entsprechend kann für den vorzugsweise einstückig gebildeten Platzhalter ein stabiler fester, insbesondere bei den vorgesehenen Einsatzbedingungen steifer, vorzugsweise biegesteifer Werkstoff wie beispielsweise Titan, Titanlegierungen, Kunststoffe oder dgl. eingesetzt werden. Allgemein kommen alle biokompatiblen Werkstoffe in Frage, die keine Abstoßreaktionen hervorrufen oder für den Körper belastende Auflösungserscheinungen zeigen.

Die Materialaussparungen können vorzugsweise in Form von nutenförmigen Vertiefungen oder offenen Durchbrechungen im zylinderrohrartigen Körperteil vorgesehen werden. Die Form, Anzahl und Anordnung der Materialaussparungen kann im Einzelfall auf die Beanspruchungsanforderungen angepasst werden.

Als eine universelle Form, die vielfältigen Anspruchsanforderungen genügt, kann die Materialaussparung insbesondere spiralförmig umlaufend um den zylinderrohrartigen Körper vorgesehen sein, so dass sich insbesondere die Form einer Art Schraubenfeder ergibt, wobei hier insbesondere vorteilhaft ist, dass durch die Materialaussparung Freiräume zwischen benachbarten Stegen des Schraubenfederelements vorliegen. Dies hat neben der leichteren Herstellbarkeit und der damit verbundenen größeren Materialauswahl auch den Vorteil, dass eine größere Flexibilität erreicht wird.

Besonders vorteilhaft können zwei Materialaussparungen vorgesehen werden, die doppelläufig bzw. 2-gängig spiralförmig ausgebildet sind. Auf diese Weise können insbesondere zwei ineinander angeordnete Schraubenfedern gebildet werden. Bei gleicher Höhe des Bereichs der spiralförmigen Aussparung können so statt einer spiralförmigen Aussparung mit niedriger Steigung zwei spiralförmige Aussparungen mit doppelter Steigung vorgesehen werden.

Die Mittel zum Verbinden des zylinderrohrartigen Körpers mit benachbarten Körperteilen können entweder einstückig mit dem zylinderrohrartigen Körper insbesondere in Verlängerung dieses Körpers an den Stirnseiten oder lösbar an den Stirnseiten angeordnet sein, wie z. B. an Endplatten, die an den Stirnseiten des zylinderrohrartigen Körpers aufgeschraubt werden können.

Derartige abnehmbare oder einstückig mit dem zylinderrohrartigen Körper verbundene Endplatten sind vorzugsweise dann vorgesehen, wenn um den zylinderrohrartigen Körper mit den Materialaussparungen zur Erzielung der Elastizität bzw. Beweglichkeit mindestens eine Hülse aus elastischem Material angeordnet ist oder innerhalb des zylinderrohrartigen Körpers mindestens ein elastischer Kern vorgesehen ist. Ein derartiger elastischer Kern oder eine elastische Hülse aus vorzugsweise einem Elastomer bietet den Vorteil, dass damit die Elastizität bzw. Steifigkeit des zylinderrohrartigen Körpers bzw. Platzhalters genau abgestimmt werden kann. Durch die modulartige Anordnung aus zylinderrohrartigem Körper mit entsprechenden Ausnehmungen sowie Kern und/oder Hülse kann durch Verwendung unterschiedlicher Komponenten mit verschiedenen Steifigkeiten in einfacher Weise eine exakt definierte Steifigkeit im Sinne von Dämpfung des Platzhalters eingestellt werden. Insofern ist ganz allgemein eine Kombination eines Platzhalterteils mit Materialaussparungen zur Erzielung einer Flexibilität und eines aus einem flexiblen Material bestehenden Platzhalterteils zur Einstellung einer definierten Steifigkeit Gegenstand der vorliegenden Erfindung. Zur Erzielung einer veränderten Steifigkeit muss lediglich die Zusammenstellung der Komponenten verändert werden, also beispielsweise muss ein anderer Kern mit einer anderen Steifigkeit oder eine andere Hülse mit dem flexiblen zylinderrohrartigen Körper verwendet werden. Obwohl es denkbar ist, gleichzeitig eine Hülse und einen Kern zusammen mit einem flexiblen zylinderrohrartigen Körper einzusetzen, wird es der Einfachheit halber meist nur eine Kombination aus zylinderrohrartigem Körper und Kern oder zylinderrohrartigem Körper und Hülse sein. Die Hülse bietet hierbei noch den Vorteil, dass sie den zylinderrohrartigen Körper mit den vorzugsweise spiralförmigen Aussparungen vor äußeren Einflüssen schützt, während dem gegenüber beim Einsatz eines Kerns dieser durch den zylinderrohrartigen Körper geschützt ist.

Sowohl Kern als auch Hülse können in vorteilhafter Weise durch die Anordnung von Endplatten an den Stirnseiten des zylinderrohrartigen Körpers gehalten werden, wobei im Falle der Anordnung einer Hülse die Endplatten über den zylinderrohrartigen Körper hinausstehen müssen und somit einen größeren Durchmesser aufweisen als der zylinderrohrartige Körper. Die Endplatten können zumindest teilweise, also auf einer Seite einstückig mit dem zylinderrohrartigem Körper verbunden sein, so dass sich hier eine becherartige Form ergibt. Darüber hinaus können die Endplatten entweder einseitig oder zweiseitig, lösbar mit dem zylinderrohrartigen Körper verbunden sein, beispielsweise über eine Schraub- oder Gewindeverbindung. Hierbei kann das Außengewinde sowohl an der Endplatte als auch am zylinderrohrartigen Körper vorgesehen sein.

Vorzugsweise ist der Platzhalter bzw. der zylinderrohrartige Körper mit den Materialaussparungen zur Erzielung von Flexibilität und Beweglichkeit in seiner Längsrichtung entlang der Platzhalterlängsachse um 0,5 bis 20 %, insbesondere 1 bis 15 % dehn- oder komprimierbar, und um eine radiale Achse senkrecht zur Platzhalterlängsachse bzw. um die Platzhalterlängsachse biegbar bzw. tordierbar, so dass die benachbarten Körperteile um ca. 0,5° bis 10°, insbesondere 1° bis 6° Grad bezüglich der Längsachse verschwenkbar oder um 0,5° bis 5° tordierbar sind.

Weitere Vorteile, Kennzeichen und Merkmale der vorliegenden Erfindung werden bei der nachfolgenden detaillierten Beschreibung zweier Ausführungsbeispiele an Hand der beigefügten Zeichnungen deutlich. Die Zeichnungen zeigen dabei in rein schematischer Weise in
- Fig. 1: eine perspektivische Ansicht eines Platzhalters für Wirbel oder Bandscheiben;
- Fig. 2: eine Seitenansicht des Platzhalters aus Fig. 1;
- Fig. 3: eine detaillierte Seitenansicht des Platzhalters aus den Fig. 1 und 2;
- Fig. 4 a) - c): Ansichten eines weiteren Platzhalters; und in
- Fig. 5 a) - c): Ansichten eines dritten Platzhalters.

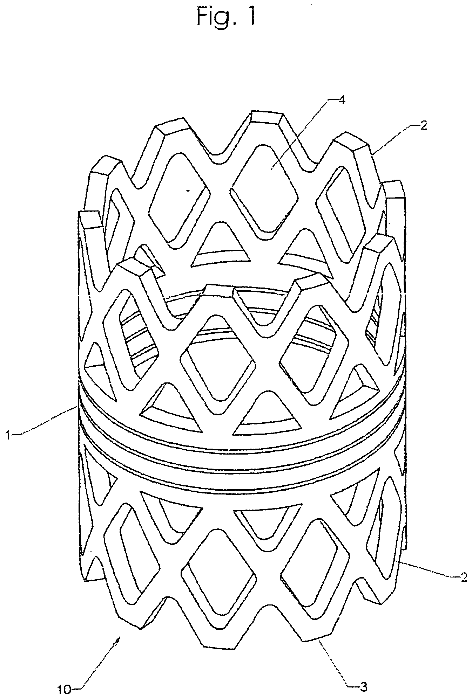

Die Fig. 1 zeigt in einer perspektivischen Darstellung eine erste Ausführungsform eines erfindungsgemäßen Implantats in Form eines Platzhalters für Wirbel oder Bandscheiben. Der Platzhalter 10 weist einen zylindrischen Körper 1 und zwei stirnseitig an dem zylindrischen Körper 1 vorgesehene Verbindungselemente 2 zum Verbinden des Platzhalters 10 mit benachbarten Körperteilen, z. B. Knochen oder Knorpel in beispielsweise einem menschlichen Körper, auf.

Die Verbindungselemente 2, die an den stirnseitigen Enden des zylinderförmigen Körpers 1 angeordnet sind, sind in dem gezeigten Ausführungsbeispiel identisch ausgebildet, können jedoch auch unterschiedlich ausgebildet sein. Die Verbindungselemente 2 weisen an ihren Stirnseiten wieder jeweils am freien Ende Zacken 3 auf, die in das benachbarte Körpergewebe am Implantationsort eingreifen können.

Die Zacken 3 sind durch dreieckförmige Ausnehmungen 5 an den beiden Stirnseiten des Platzhalters 10 gebildet, so dass sich trapezförmige Zacken 3 bilden, die in benachbartes Körpergewebe eingreifen und sich mit diesem verkrallen können.

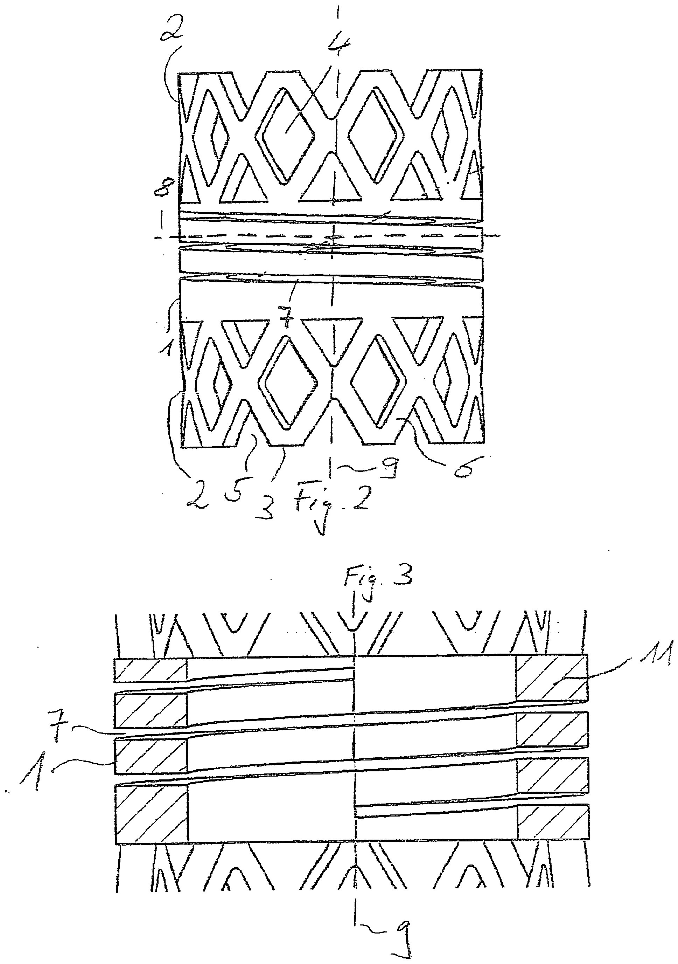

Darüber hinaus weisen die Verbindungselemente 2 rautenförmige Ausnehmungen 4 auf (siehe Fig. 2), die benachbart zueinander um die gesamte Zylindermantelfläche der Verbindungselemente 2 vorgesehen sind. Dadurch ist das jeweilige Verbindungselement an sich selbst wieder durch eine Vielzahl von rautenförmig miteinander verbundenen Stegen 6 gebildet, wobei die Spitzen der durch die Stege 6 gebildeten Rauten abgeschnitten sind, um die trapezförmigen Zacken 3 zu bilden.

Der zylinderrohrartige Körper 1 zwischen den Verbindungselementen 2 an den jeweiligen Stirnseiten des Zylinders weist in dem gezeigten Ausführungsbeispiel eine spiralförmige Materialaussparung 7 auf, so dass die Wandung 11 (siehe Fig. 3) selbst eine Spiralform annimmt. Da ansonsten der Platzhalter 10 insgesamt als Hohlzylinder ausgebildet ist, stellt der zylinderrohrartige Körper 1 zwischen den Verbindungselementen 2 mit der Materialaussparung 7 einen elastischen Bereich bzw. einen Bewegungsbereich dar, selbst wenn der Platzhalter 10 selbst aus einem im wesentlichen steifen Material, wie z. B. Titan oder einer Titanlegierung, gebildet ist. Durch die Materialaussparung 7 erhält der Platzhalter 10 eine konstruktiv bedingte Elastizität im Bereich des zylinderrohrartigen Körpers 1, die es entbehrlich macht, in diesem Bereich zur Erzielung einer gewissen Elastizität bzw. Beweglichkeit ein gesondertes elastisches Material zur Verfügung zu stellen. Insbesondere kann damit vermieden werden, den Platzhalter aus mehreren zusammenzufügenden Teilen herstellen zu müssen.

Durch die spiralförmige Materialaussparung 7 wird in einfacher Weise eine Dehnbarkeit und Kompressibilität des zylinderrohrartigen Körpers 1 entlang der Längsachse 9 des Platzhalters 10 sowie eine Biegbarkeit um eine Drehachse senkrecht zur Längsachse 9, welche beispielsweise durch die Achse 8 verdeutlicht ist (Fig. 2), erreicht. Hier hat sich insbesondere die Spiralform der Materialaussparung 7 bewährt, die eine ausgewogene Elastizität bzw. Beweglichkeit in unterschiedlichste Richtungen ermöglicht. Selbstverständlich sind jedoch auch andere Formen von Materialaussparungen sowie eine unterschiedliche Anzahl und Anordnung dieser Materialaussparungen möglich und denkbar, wobei auf den Einzelfall bzw. das Beanspruchungsprofil des Einzelfalls angepasste Lösungen möglich sind.

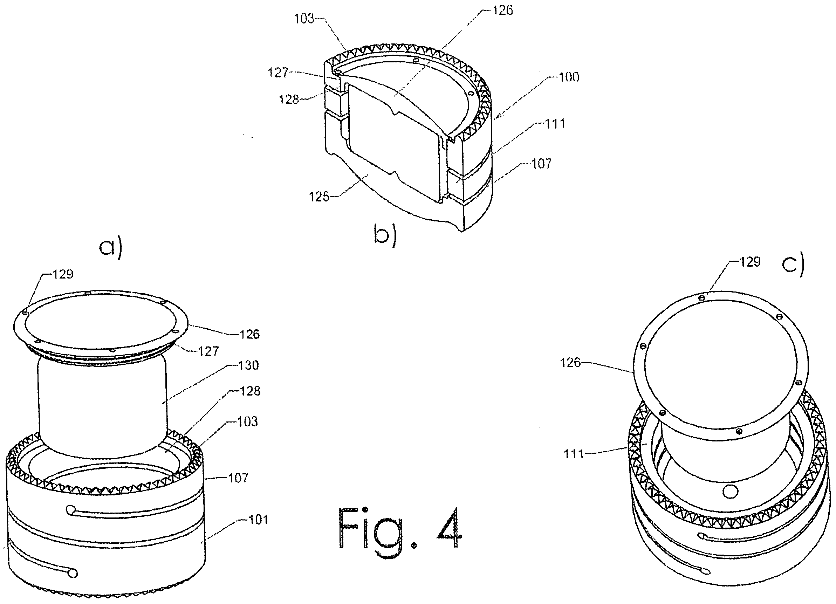

Die Fig. 4 zeigt in den Teilbildern a) bis c) drei verschiedene perspektivische Explosionsdarstellungen sowie Schnittansichten (b) einer zweiten Ausführungsform eines Platzhalters 100 mit einem zylinderrohrartigen Körper 101, der an der Unterseite durch eine einstückig mit dem zylinderrohrartigen Körper 101 verbundene Endplatte 125 verschlossen ist, so dass sich eine becherartige Form ergibt.

Der zylinderrohrartige Körper 101 weist in seiner Wandung 111 eine spiralförmig verlaufende Ausnehmung 107 auf, die dem zylinderrohrartigen Körper 101 erfindungsgemäß eine gewisse Flexibilität verleiht.

Um die Steifigkeit des Platzhalters 100 genau anpassen zu können, ist in dem zylinderrohrartigen Körper 101 ein austauschbares Kernelement 130 aus einem elastomeren Werkstoff vorgesehen, welches an der Unterseite über die Endplatte 125 und an der Oberseite über die Endplatte 126 in dem zylinderrohrartigen Körper 101 gehalten ist.

Die Endplatte 126 an der Oberseite des Platzhalters weist ein Außengewinde 127 auf, mittels dem es in das Innengewinde 128 des zylinderrohrartigen Körpers 101 an der oberen Stirnfläche in der Innenseite des zylinderrohrartigen Körpers 101 eingeschraubt werden kann. Die Endplatte 126 weist eine Schulter auf, mit der sie auf der Wandung 111 dicht aufliegt. Umlaufend sind an der Stirnseite an der Wandung 111 Zacken 103 vorgesehen, die über die Endplatten 125 und 126 hervorstehen und in das benachbarte Gewebe eingreifen können, um somit den Platzhalter an Ort und Stelle festzuhalten.

Die Endplatte 126 weist Eingriffsöffnungen 129 auf, mittels der die Endplatte 126 in den zylinderrohrartigen Körper 101 eingeschraubt werden kann. Die Endplatten 125 und 126 können zudem an der Außenseite aufgerauht oder bioaktiv beschichtet sein, so dass das Einwachsen unterstützt wird.

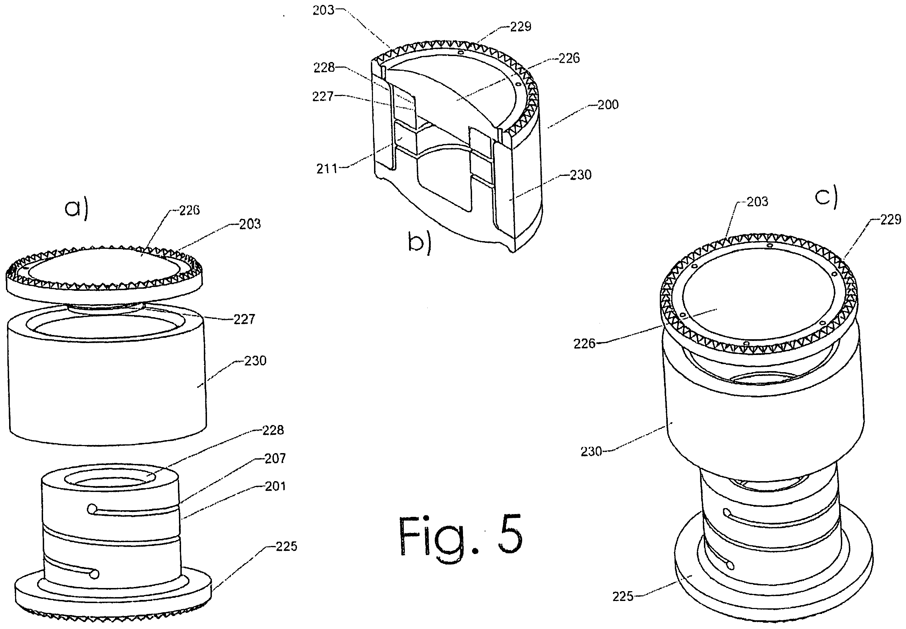

Die Fig. 5 zeigt in den Teilbildern a) bis c) eine dritte Ausführungsform eines Platzhalters, wobei die Teilbilder a) bis c) perspektivische Explosionsdarstellungen darstellen, während Teilbild b) eine perspektivische Schnittansicht zeigt. Bei der Ausführungsform der Fig. 5 weist der zylinderrohrartige Körper 201 wiederum eine spiralförmige Ausnehmung 207 in der Körperwandung 211 auf.

An der Unterseite ist der zylinderrohrartige Körper 201 wieder über eine einstückig angeordnete Endplatte 225 verschlossen, so dass sich auch hier eine becherartige Form des zylinderrohrartigen Körpers 201 ergibt. Allerdings ist die Endplatte 225 so ausgebildet, dass sie einen größeren Außendurchmesser aufweist als der zylinderrohrartige Körper 201, in dem die spiralförmige Materialausnehmung 207 angeordnet ist. Somit entsteht eine Schulter, die eine Aufnahme für eine rohrartige Hülse 230 aus Elastomermaterial bildet. Die elastische Hülse 230 wird über den zylinderrohrartigen Körper 201 geschoben, so dass dieser vollständig von der Hülse umgeben ist. An der oberen Stirnfläche wird eine Endplatte 226 mittels einer Gewindeverbindung an den zylinderrohrartigen Körper 201 angeschraubt. Hierbei greift das Außengewinde 227 der Endplatte 226 in das Innengewinde 228 des zylinderrohrartigen Körpers 201 ein, so dass die Hülse 230 zwischen den Endplatten 225 und 226 festgehalten wird. Auch die Hülse 230 dient zur Anpassung der Gesamtsteifigkeit, wobei durch ein einfaches Auswechseln der Hülse 230 in ähnlicher Weise wie durch ein Austauschen des Kerns 130 (siehe Fig. 4) eine einfache Variation der Steifigkeit des Gesamtimplantats 100 bzw. 200 möglich ist.

An den Endplatten 226 bzw. 225 sind pyramidenförmige Zacken 203 vorgesehen, die zum Eingreifen in das benachbarte Gewebe dienen, um so den Platzhalter fest zu verankern. Auch die Endplatten 225 und 226 können zumindest an ihren Außenseiten aufgerauht oder bioaktiv beschichtet sein.

Auch der Deckel 226 weist Eingriffsöffnungen 229 auf, mittels derer die Endplatte 226 auf den zylinderrohrartigen Körper 201 aufgeschraubt werden kann.

## Patentansprüche

1. Platzhalter für Wirbel und/oder Bandscheiben mit Platzhalter- und Gewichtsübertragungsfunktion zur zeitweisen oder dauerhaften Verbringung in einen menschlichen oder tierischen Körper aus mindestens einem biokompatiblen Werkstoff mit einem zylinderrohrartigen Körper (1) und mit an den stirnseitigen Enden des zylinderrohrartigen Körpers vorgesehenen Mitteln (2) zum Verbinden mit benachbarten Körperteilen, wobei im zylinderrohrartigen Körper Materialaussparungen vorgesehen sind, die zur lokalen Reduzierung der Steifigkeit dienen,
wobei der zylinderrohrartige Körper (1) von einer aus einem Elastomer bestehenden Hülse umgeben oder/und mit einem aus einem Elastomer bestehenden Kernelement versehen ist, welche durch an dem zylinderrohrartigen Körper einstückig und/oder lösbar, insbesondere über eine Schraub- oder Gewindeverbindung, angeordnete Endplatten gehalten werden und welche mit dem zylinderrohrartigen Körper mit Materialaussparungen zur Erzielung von Flexibilität zusammen wirken, so dass eine definierte Steifigkeit oder Beweglichkeit des Gesamtimplantats eingestellt werden kann, so dass der Platzhalter in axialer Richtung komprimier- und dehnbar und bezüglich der stirnseitig vorgesehenen Mittel (2) zum Verbinden des Platzhalters mit benachbarten Körperteilen um eine radiale Drehachse (8) biegbar und um eine axiale Drehachse tordierbar ist.

2. Platzhalter nach Anspruch 1,
**dadurch gekennzeichnet, dass**
der zylinderrohrartige Körper und die Mittel zum Verbinden mit benachbarten Körperteilen einstückig aus einem Werkstoff gebildet sind.

3. Platzhalter nach einem der vorhergehenden Ansprüche,
**dadurch gekennzeichnet, dass**
der biokompatible Werkstoff insbesondere des zylinderrohrartigen Körpers aus einem bei den vorgesehenen Einsatzbedingungen steifen, insbesondere biegesteifen Material ist.

4. Platzhalter nach einem der vorhergehenden Ansprüche,
**dadurch gekennzeichnet, dass**
der biokompatible Werkstoff aus der Gruppe ausgewählt ist, die Titan und Legierungen davon sowie Kunststoffe umfasst.

5. Platzhalter nach einem der vorhergehenden Ansprüche,
**dadurch gekennzeichnet, dass**
die Materialaussparung (7, 19) als nutenförmige Vertiefung und/oder als offene Durchbrechung, insbesondere spiralförmig ausgebildet ist.

6. Platzhalter nach einem der vorhergehenden Ansprüche,
**dadurch gekennzeichnet, dass**
zwei Materialaussparungen als nutenförmige Vertiefung und/oder als offene Durchbrechung doppelläufig spiralförmig ineinander angeordnet sind.

7. Platzhalter nach einem der vorhergehenden Ansprüche,
**dadurch gekennzeichnet, dass**
die Mittel (2) zum Verbinden des zylinderrohrartigen Körpers mit benachbarten Körperteilen Vorsprünge (3), insbesondere in axialer Richtung des zylinderrohrartigen Körpers sich erstreckende Zacken (3) mit insbesondere dreiecks- und/oder trapezförmiger Gestalt zum Eindringen in benachbarte Körperteile, insbesondere Knochen, Knorpel und dergleichen aufweisen.

8. Platzhalter nach einem der vorhergehenden Ansprüche,
**dadurch gekennzeichnet, dass**
die Mittel (2) zum Verbinden des zylinderrohrartigen Körpers mit benachbarten Körperteilen als vorzugsweise einstückige Verlängerung des zylinderrohrartigen Körper (1) ausgebildet sind, wobei Vertiefungen, Öffnungen und/oder Aussparungen (4) in der Mantelfläche zum Einwachsen vorgesehen sind.

9. Platzhalter nach einem der vorhergehenden Ansprüche,
**dadurch gekennzeichnet, dass**
die Mittel (2) zum Verbinden des zylinderrohrartigen Körpers mit benachbarten Körperteilen an einer am zylinderrohrartigen Körper lösbar an der Stirnseite vorgesehenen Endplatte angeordnet sind.

10. Platzhalter nach einem der vorhergehenden Ansprüche,
**dadurch gekennzeichnet, dass**
der Platzhalter und insbesondere der zylinderrohrartige Körper bezogen auf seine Längsrichtung um 0,5 bis 20 %, insbesondere 1 bis 15 % elastisch dehn- oder komprimierbar ist.

11. Platzhalter nach einem der vorhergehenden Ansprüche,
**dadurch gekennzeichnet, dass**
der Platzhalter und insbesondere der zylinderrohrartige Körper (1) um eine radiale Achse (3) elastisch biegbar ist, so dass die stirnseitig vorgesehenen Mittel (2) zum Verbinden mit benachbarten Körperteilen um ca. 0,5° bis 10 °, insbesondere 1° bis 6 ° aus der Längsachse (12) des zylinderrohrartigen Körpers verschwenkbar sind oder dass der Platzhalter und insbesondere der zylinderrohrartige Körper um eine axiale Achse elastisch tordierbar ist, so dass die stirnseitig vorgesehenen Mittel (2) zum Verbinden mit benachbarten Körperteilen um ca. 0,5° bis 5°, insbesondere 1° bis 3° verdrehbar ist.

## Claims

1. A space holder for vertebrae and/or intervertebral discs with space-holding and weight-transfer function for temporary or permanent introduction into a human or animal body and made from at least one biocompatible material having a tube-like body (1) and having, on the end-faces of the tube-like body, means (2) for connecting to adjacent body parts wherein the tube-like body has recesses to locally reduce the rigidity, wherein the tube-like body (1) is surrounded by a sleeve made from an elastomer and/or is provided with a core made from an elastomer, which are held by end plates which are disposed monolithically and/or detachably, especially by means of a screw or threaded connection, on the tube-like body and which cooperate with the tube-like body with recesses for the purpose of achieving flexibility, such that the desired frigidity or mobility of the overall implant can be adjusted, such that the space holder is compressible and extensible in the axial direction and, with respect to the means (2) provided on the end-faces for the purpose of connecting the space holder to adjacent body parts, can be bent about a radial rotational axis (13) and twisted about an axial rotational axis.

2. Space holder in accordance with claim 1,
**characterised in that**
the tube-like body and the means for connecting to adjacent body parts are formed monolithically from one material.

3. Space holder in accordance with any of the preceding claims
**characterised in that**
the biocompatible material, especially of the tube-like body, is made from a material which is stiff, especially rigid, in the intended application conditions.

4. Space holder in accordance with any of the preceding claims
**characterised in that**
the biocompatible material is selected from the group comprising titanium, titanium alloys, and polymers.

5. Space holder in accordance with any of the preceding claims,
**characterised in that**
the recess (7, 19) is a groove and/or an open aperture, and especially is helical.

6. Space holder in accordance with any of the preceding claims,
**characterised in that**
two recesses as a groove-like depression and/or as an open aperture are arranged in a double-helix in each other.

7. Space holder in accordance with any of the preceding claims,
**characterised in that**
the means (2) for connecting the tube-like body to adjacent body parts have projections (3), especially serrations (3) extending in the axial direction of the tube-like body with especially a triangular and/or trapezoidal shape for the purpose of penetrating into adjacent body parts, especially bones, cartilage and the like.

8. Space holder in accordance with any of the preceding claims,
**characterised in that**
the means (2) for connecting the tube-like body to adjacent body parts are formed as a preferably monolithic extension of the tube-like body (1), wherein depressions, openings and/or recesses (4) are disposed in the lateral surface for the purpose of in-growth.

9. Space holder in accordance with any of the preceding claims,
**characterised in that**
the means (2) for connecting the tube-like body to adjacent body parts are disposed on an end plate which is detachably disposed on the end-face of the tube-like body.

10. Space holder in accordance with any of the preceding claims,
**characterised in that**
the space holder and especially the tube-like body is elastically extensible or compressible by 0.5 to 20%, especially 1 to 15%, with respect to its longitudinal direction.

11. Space holder in accordance with any of the preceding claims,
**characterised in that**
the space holder and especially the tube-like body (1) are elastically bendable about a radial axis (3), such that the means (2) provided on the end-faces for the purpose of connecting to adjacent body parts can be pivoted by approx. 0.5° to 10°, especially 1° to 6°, out of the longitudinal axis (12) of the tube-like body or that the space holder and especially the tube-like body can be twisted elastically about an axial axis, such that the means (2) provided on the end faces for connecting to adjacent body parts can be rotated by approx. 0.5° to 5°, especially 1° to 3°.

## Revendications

1. Dispositif destiné à se mettre à la place de vertèbres et /ou de disques intervertébraux avec fonction de maintien d'espace et de transfert de poids pour l'implantation temporaire ou durable dans un corps humain ou animal composé d'au moins un matériau biocompatible avec un corps de type tube de cylindre (1) et avec des moyens (2) prévus aux extrémités frontales du corps de type tube de cylindre pour être reliés avec des parties de corps voisines, cependant que des évidements de matériau sont prévus dans le corps de type tube de cylindre qui servent à la réduction locale de la rigidité, cependant que le corps de type tube de cylindre (1) est entouré par une gaine constituée par un élastomère et/ou est pourvu d'un élément de noyau constitué par un élastomère, qui sont maintenus par des plaques d'extrémité placées en une pièce sur le corps de type tube de cylindre et/ou amovibles, en particulier par un assemblage vissé ou fileté et qui coopèrent avec le corps de type tube de cylindre avec des évidements de matériau pour obtenir la flexibilité si bien qu'une rigidité ou une mobilité définie de l'ensemble de l'implant peut être ajustée si bien que le dispositif destiné à se mettre à la place de vertèbres et /ou de disques intervertébraux peut être comprimé et dilaté dans le sens axial et peut être courbé autour d'un axe de rotation radial (8) par rapport aux moyens (2) prévus sur le côté frontal pour relier le dispositif destiné à se mettre à la place de vertèbres et /ou de disques intervertébraux à des parties de corps voisines et peut être tordu autour d'un axe de rotation axial.

2. Dispositif destiné à se mettre à la place de vertèbres et /ou de disques intervertébraux selon la revendication 1, **caractérisé en ce que** le corps de type tube de cylindre et les moyens pour être reliés à des parties de corps voisines sont formés en une pièce en un matériau.

3. Dispositif destiné à se mettre à la place de vertèbres et /ou de disques intervertébraux selon l'une des revendications précédentes, **caractérisé en ce que** le matériau biocompatible en particulier du corps de type tube de cylindre est en un matériau rigide pour les conditions d'utilisation prévues, en particulier rigide à la flexion.

4. Dispositif destiné à se mettre à la place de vertèbres et /ou de disques intervertébraux selon l'une des revendications précédentes, **caractérisé en ce que** le matériau biocompatible est sélectionné dans le groupe qui comprend le titane et les alliages de celui-ci ainsi que des matières synthétiques.

5. Dispositif destiné à se mettre à la place de vertèbres et /ou de disques intervertébraux selon l'une des revendications précédentes, **caractérisé en ce que** l'évidement de matériau (7, 19) est configuré comme un approfondissement en forme de rainure et/ou comme une découpure ouverte en forme de spirale.

6. Dispositif destiné à se mettre à la place de vertèbres et /ou de disques intervertébraux selon l'une des revendications précédentes, **caractérisé en ce que** deux évidements de matériau sont placés l'un dans l'autre comme approfondissement en forme de rainure et/ou comme découpure ouverte en forme de spirale double.

7. Dispositif destiné à se mettre à la place de vertèbres et /ou de disques intervertébraux selon l'une des revendications précédentes, **caractérisé en ce que** les moyens (2) pour relier le corps de type tube de cylindre à des parties de corps voisines présentent des saillies (3), en particulier des dents (3) qui s'étendent dans le sens axial du corps de type tube de cylindre avec une configuration en particulier de forme triangulaire et/ou trapézoïdale pour pénétrer dans des parties de corps voisines, en particulier des os, du cartilage et équivalent.

8. Dispositif destiné à se mettre à la place de vertèbres et /ou de disques intervertébraux selon l'une des revendications précédentes, **caractérisé en ce que** les moyens (2) pour relier le corps de type tube de cylindre à des parties de corps voisines sont configurés comme un prolongement en une pièce du corps de type tube de cylindre (1), cependant que des ouvertures et/ou des évidements (4) sont prévus dans la surface d'enveloppe pour la croissance intérieure.

9. Dispositif destiné à se mettre à la place de vertèbres et /ou de disques intervertébraux selon l'une des revendications précédentes, **caractérisé en ce que** les moyens (2) pour relier le corps de type tube de cylindre à des parties de corps voisines sont placés sur une plaque d'extrémité prévue sur le corps de type tube de cylindre sur le côté frontal en étant amovible.

10. Dispositif destiné à se mettre à la place de vertèbres et /ou de disques intervertébraux selon l'une des revendications précédentes, **caractérisé en ce que** le dispositif destiné à se mettre à la place de vertèbres et /ou de disques intervertébraux et en particulier le corps de type tube de cylindre peut être dilaté ou comprimé élastiquement par rapport à son sens longitudinal de 0,5 à 20%, en particulier de 1 à 15%.

11. Dispositif destiné à se mettre à la place de vertèbres et /ou de disques intervertébraux selon l'une des revendications précédentes, **caractérisé en ce que** le dispositif destiné à se mettre à la place de vertèbres et /ou de disques intervertébraux et en particulier le corps de type tube de cylindre peut être courbé élastiquement si bien que les moyens prévus sur le côté frontal (2) pour être reliés à des parties de corps voisines sont pivotants d'environ 0,5° à 10°, en particulier de 1° à 6° à partir de l'axe longitudinal (12) du corps de type tube de cylindre ou que le dispositif destiné à se mettre à la place de vertèbres et /ou de disques intervertébraux et en particulier le corps de type tube de cylindre peut être tordu élastiquement autour d'un axe axial si bien que les moyens prévus sur le côté frontal (2) pour être reliés à des parties de corps voisines peuvent tourner d'environ 0,5° à 5°, en particulier de 1° à 3°.
